# EUROPÄISCHE PATENTSCHRIFT

(11) **EP 0 733 611 B1**
(45) Veröffentlichungstag und Bekanntmachung des Hinweises auf die Patenterteilung: **19.07.2000**
(21) Anmeldenummer: 96103774.4
(22) Anmeldetag: 11.03.1996
(51) Int. Cl.: C07C 17/21, C07C 19/08

(54) **Verfahren zur Herstellung von Pentafluorethan (R 125)**
Process for the preparation of pentafluorethane (R 125)
Procédé pour la préparation de pentafluoroéthane (R 125)

(30) Priorität: 20.03.1995 DE 19510024
(43) Veröffentlichungstag der Anmeldung: 25.09.1996
(73) Patentinhaber: SOLVAY (Société Anonyme), 1050 Bruxelles (BE)
(72) Erfinder: Deger, Hans Matthias, Dr., D-65719 Hofheim (DE); Krause, Werner, Dr., D-50354 Hürth (DE); Schmid, Dieter, Dr., D-65824 Schwalbach (DE); Hoeveler, Martin, Dr., D-65510 Idstein (DE)
(74) Vertreter: Jacques, Philippe

(56) Entgegenhaltungen:
- EP-A- 0 130 532
- EP-A- 0 407 961
- DE-A- 2 032 098
- US-A- 3 258 500
- CHEMICAL ABSTRACTS, vol. 113, no. 17, 22.Oktober 1990 Columbus, Ohio, US; abstract no. 151815, MORIKAWA S ET AL: "Fluorination of perchloroethylene" XP002006782 & JP-A-02 178 237 (ASAHI GLASS CO., LTD)

## Beschreibung

Die vorliegende Erfindung betrifft ein Verfahren zur Herstellung von Pentafluorethan (R 125) durch Umsetzung von Perchlorethylen (Cl₂C = CCl₂) mit Fluorwasserstoff in der Gasphase an einem chromhaltigen Katalysator. R 125 schädigt als chlorfreie Verbindung die Ozonschicht nicht und ist daher als Ersatzstoff für FCKW geeignet.

Derartige Verfahren zur Herstellung von R 125 sind bereits bekannt. GB-A-1 307 224 beschreibt diese Umsetzung unter Verwendung eines ungeträgerten Chromoxid Katalysators (Table IV, Example 23). Dabei werden jedoch als Nebenprodukt 20 % R 115 (CF₃CF₂Cl) gebildet, das ein hohes Ozonabbaupotential (ODP) besitzt und daher weitestgehend aus dem gewünschten R 125 entfernt werden muß. Da R 115 mit R 125 ein Azeotrop bildet, ist seine Entfernung nur mit erheblichen Aufwand und unter Verlust eines Teils des R 125 möglich. R 115 kann nicht in den Prozeß zurückgeführt werden.

US-A-3 258 500 beschreibt ebenfalls die Umsetzung von Perchlorethylen zu R 125 an einem Chromoxidkatalysator (Example 17). Auch hier ist jedoch die "Wertstoffselektivität" zu niedrig für ein wirtschaftliches Verfahren, d.h. die Selektivität zu R 125 und solchen Nebenprodukten (C₂HCl₁₊ₓ F₄₋ₓ, x = 0 bis 3) die man in das Verfahren zurückführen und so in R 125 umwandeln kann. Bei 300°C ist die Wertstoffselektivität weniger als 80 % und bei 450°C weniger als 50 %.

Bei Einsatz des in EP-A-0 609 123 beschriebenen Katalysators, der zusätzlich zu Chrom noch Nickel enthält, in der genannten Umsetzung werden mehrere tausend ppm bis 1,4 Prozent des Olefins Trichlorfluorethylen (R 1111) gebildet (Seite 6, Tabelle 1 und Seite 7, Tabelle 2). Dieses muß wegen seiner Giftigkeit entfernt werden, was aber einen erheblichen Aufwand bedeutet.

Es wurde nun gefunden, daß man mit Hilfe eines auf bestimmte Art hergestellten, Chrom und Magnesium sowie optional Graphit enthaltenden Katalysators hochreines R 125 aus Perchlorethylen und Fluorwasserstoff erhält.

Gegenstand der Erfindung ist ein Verfahren zur Herstellung von Pentafluorethan (R 125) durch Reaktion von Perchlorethylen mit Fluorwasserstoff in der Gasphase an einem chromhaltigen Katalysator, dadurch gekennzeichnet, daß ein Chrom und Magnesium enthaltender Katalysator verwendet wird, der dadurch erhältlich ist, daß man ein wasserlösliches Chrom(III)-Salz mit Magnesiumhydroxid oder Magnesiumoxid sowie optional Graphit in Gegenwart von Wasser umsetzt, die Reaktionsmischung in einen Teig überführt, dann den Teig trocknet und bei Temperaturen von 20 bis 500°C mit Fluorwasserstoff behandelt, wobei man solche Mengen an Chrom- und Magnesiumsalzen sowie optional Graphit verwendet, daß der getrocknete Teig vor der Behandlung mit Fluorwasserstoff 4,5 bis 26 Gew.-% Cr, ausgedrückt als Cr₂O₃, und mindestens 25 Gew.-% Mg, ausgedrückt als MgO, sowie optional Graphit enthält.

Durch das erfindungsgemäße Verfahren läßt sich überraschenderweise Pentafluorethan in hoher Ausbeute herstellen. Die dabei als Nebenprodukte entstehenden Verbindungen der Formel C₂HCl₁₊ₓF₄₋ₓ, x = 0 bis 3, d.h. C₂HClF₄, C₂HCl₂F₃, C₂HCl₃F₂ und C₂HCl₃F, lassen sich durch Rückführung in den Reaktor zu R 125 umsetzen; sie werden daher im folgenden auch (neben R125 selbst) als "Wertstoffe" bezeichnet.
Das Verfahren hat den großen Vorteil, daß von diesen Wertstoffen abgesehen, keine weiteren Nebenprodukte in wesentlichen Mengen entstehen.

Katalysatoren, die unter die eben gegebene Definition fallen, sind bereits in der Literatur beschrieben, werden aber dort für andere Umsetzungen benutzt:

Gemäß EP-A-0 130 532 wird ein Katalysator, bei dessen Herstellung 1 Mol eines wasserlöslichen Chrom(III)-Salzes mit mindestens 1,5 Mol, vorzugsweise 12 bis 24 Mol, Magnesiumhydroxid oder Magnesiumoxid umgesetzt wurden, zur Fluorierung oder Dismutierung von Halogenalkanen mittels HF eingesetzt (Spalte 5, Zeile 4-9, sowie die Beispiele). Dabei wird in gesättigten Halogenkohlenwasserstoffen Chlor gegen Fluor ausgetauscht. Eine Addition von HF an Perchlorethylen und ein Austausch sämtlicher Chloratome gegen Fluoratome, wie sie beim vorliegenden Verfahren eintritt, wird weder beschrieben, noch nahegelegt.

Der gleiche Katalysator wie in EP-A-0 130 532 wird gemäß EP-A-0 417 680 dazu eingesetzt, 1,1,1-Trifluor-2-chlorethan mit HF zu 1,1,1,2-Tetrafluorethan, umzusetzen. Hierbei wird wieder in einem gesättigten Halogenkohlenwasserstoff Chlor durch Fluor ersetzt.

Der gleiche Katalysator wird gemäß EP-A-0 407 961 dazu eingesetzt, 1,1,1-Trifluor-2-chlorethan durch Umsetzung von Trichlorethylen mit HF herzustellen. Hierbei handelt es sich zwar um eine Addition von HF an einen ungesättigten Halogenkohlenwasserstoff, verbunden mit einem teilweisen Austausch von Chlor gegen Fluor, jedoch werden von den drei im Ausgangsmaterial enthaltenen Chloratomen nur zwei durch Fluoratome ersetzt. Dagegen wird beim vorliegenden Verfahren nicht nur HF addiert, sondern alle vier im Ausgangsmaterial Perchlorethylen Cl₂C = CCl₂ enthaltenen Chloratome durch Fluoratome ersetzt, so daß als Endprodukt der chlorfreie Fluorkohlenwasserstoff Pentafluorethan CF₃-CF₂H (R 125) entsteht.

Die erfindungsgemäße Reaktion läuft mit nahezu quantitativem Umsatz des Perchlorethylens bei hohen Ausbeuten an R 125 ab, sowie mit hoher Selektivität für die Wertstoffe des Prozesses (R 125 und die bereits erwähnten, durch Recyclierung in R 125 umwandelbaren Nebenprodukte). Eine zwischenzeitliche Aufarbeitung des Rohgases, z.B. Abtrennung der bei der Umsetzung entstehenden HCl, um das Gleichgewicht zum R 125 zu verschieben, ist nicht nötig. Toxische Olefine wie R 1111 (Trichlorfluorethylen) und R 1112a (1,1-Dichlor-2,2-difluorethylen) entstehen nur in sehr geringen Mengen in der Nähe der Nachweisgrenze.

Der erfindungsgemäß eingesetzte Katalysator ist dadurch erhältlich, daß man ein wasserlösliches Chrom(III)-Salz mit Magnesiumhydroxid oder Magnesiumoxid sowie optional Graphit in Gegenwart von Wasser umsetzt, die Reaktionsmischung in einen Teig überführt, dann den Teig trocknet, und bei Temperaturen von 20 bis 500°C mit Fluorwasserstoff behandelt, wobei man solche Mengen an Chrom- und Magnesiumsalzen sowie optional Graphit verwendet, daß der getrocknete Teig vor der Behandlung mit Fluorwasserstoff 4,5 bis 26 Gew.-% Cr, ausgedrückt als Cr₂O₃, und mindestens 25 Gew.-% Mg, ausgedrückt als MgO, sowie optional Graphit enthält.

Vorzugsweise verwendet man solche Mengen an Chrom- und Magnesiumsalzen sowie optional Graphit, daß der getrocknete Teig vor der Behandlung mit Fluorwasserstoff 5,5 bis 23 Gew.-% Cr, ausgedrückt als Cr₂O₃, und mindestens 25 Gew.-% Mg, ausgedrückt als MgO, sowie optional Graphit enthält. Der Anteil an Graphit, welcher nicht katalytisch aktiv ist, sondern die Formung der Katalysatorteilchen unterstützt, beträgt vorzugsweise 5 bis 40 Gew.-%.

Die Bedeutung der Ausdrücke "Cr, ausgedrückt als Cr₂O₃" und "Mg, ausgedrückt als MgO" soll an folgendem konkreten Beispiel einer Berechnung erläutert werden:
Die angegebenen Prozentgehalte beziehen sich auf einen Zeitpunkt nach der Trocknung und vor der HF-Behandlung des Katalysators. Chrom und Magnesium werden als Cr₂O₃ und MgO ausgedrückt, um die Abhängigkeit der Prozentberechnung von den für die Reaktion irrelevanten Anionen der jeweils gewählten Chrom- und Magnesiumsalze zu eliminieren.
Zur Herstellung eines für das erfindungsgemäße Verfahren geeigneten Katalysators werden beispielsweise 2800 g Cr(NO₃)₃ • 9 H₂O, 2000 g MgO, 800 g Graphit und 4000 g Wasser eingesetzt. Die angegebene Menge an Chromsalz entspricht, als Cr₂O₃ gerechnet, einer Menge von 531,8 g. Der Gehalt dieses Katalysators an Chrom, ausgedrückt als Cr₂O₃, errechnet sich wie folgt: 531.8 g (Cr₂O₃) / (531.8 g (Cr₂O₃) + 2000 g (MgO) + 800 g (Graphit)) = 531.8 g/3331.8 g = 16 Gew.-%. Der Gehalt an Magnesium, ausgedrückt als MgO, beträgt 2000 g/3331.8 g = 60 % Gew.-%, und der Graphitgehalt beträgt 800 g/3331.8 g = 24 Gew.-%.

Als Chrom(III)-Verbindung können sowohl wasserfreie als auch - was bevorzugt ist - die hydratisierten Salze des dreiwertigen Chroms eingesetzt werden, die leicht zugänglich und kommerziell erhältlich sind. Verwendbar sind beispielsweise Chrom(III)-sulfat und Chrom(III)-fluorid, vorzugsweise aber Chrom(III)-chlorid und Chrom(III)-nitrat.

Das eingesetzte Magnesiumoxid darf nicht geglüht sein, sondern muß noch mit schwach sauren Verbindungen reagieren können. Da bei der Herstellung des Katalysators kein Auswaschvorgang eingeschaltet wird, ist das Molverhältnis Magnesium/Chrom der Einsatzkomponenten dasselbe wie im fertigen Katalysator.

Die eingesetzte Wassermenge ist für die Katalysatorherstellung nicht kritisch; die Wassermenge muß jedoch ausreichen, damit die Masse zumindest durch einen Kneter verarbeitbar ist. Je höher die eingesetzte Wassermenge, umso mehr Wasser muß jedoch bei der Trocknung verdampft werden.

Zur Herstellung der Katalysatoren kann man beispielsweise die Chromverbindung als wäßrige Lösung zu trockenem Magnesiumoxid oder -hydroxid geben und die resultierende Mischung, optional zusammen mit Graphit, zu einer Paste verkneten.

Man kann auch stattdessen das vorgelegte Magnesiumoxid mit Wasser anteigen, die Chromverbindung trocken zugeben, optional Graphit hinzufügen, und die Reaktionsmischung verkneten.

Das Verkneten erfolgt vorteilhafterweise mit Maschinen, die in der Verfahrenstechnik üblicherweise zum Vermischen pastöser Stoffe dienen, z.B. Vertikal-Kneter oder Duplex-Kneter.

Bei der Herstellung der erfindungsgemäß eingesetzten Katalysatoren wird eine Paste erhalten, die ohne Waschen getrocknet wird. Sie ist direkt zur Herstellung von Formkörpern geeignet. Von Vorteil ist es, daß die Formkörper mit den üblichen verfahrenstechnischen Maßnahmen, wie z.B. Pelletisieren, Extrudieren oder Granulieren hergestellt werden können.

Nach der Formgebung erfolgt die Trocknung der Formkörper, die mechanisch sehr stabile Katalysatorteilchen ergibt. Die Trocknung kann sowohl bei Raumtemperatur als auch bei erhöhter Temperatur erfolgen. Zweckmäßigerweise wird eine Trocknungstemperatur von 50° bis 150°C gewählt, vorzugsweise 70° bis 120°C, um die Trocknungszeit kurz zu halten. Die Trocknung kann sowohl unter Normaldruck als auch unter Vakuum erfolgen.

Die Trocknung sollte nicht bei Temperaturen über 400°C erfolgen, da sonst das Chromoxid seine Reaktionsfähigkeit gegenüber Fluorwasserstoff verlieren kann.

Die Behandlung des Katalysators mit Fluorwasserstoff vor seinem Einsatz erfolgt zweckmäßigerweise bei Temperaturen von 150 bis 500°C, vorzugsweise 150 bis 450°C. Die eingesetzte Menge an Fluorwasserstoff ist nicht kritisch. Die Fluorierungszeit kann in weiten Grenzen gewählt werden; bevorzugt sind 0,5 bis 10 Stunden. Um das entstehende Wasser schnell zu entfernen und unerwünschte Temperaturspitzen zu vermeiden, wird HF vorzugsweise durch ein Inertgas (z.B. N₂ oder Luft) verdünnt.

Bei späterem Abfall der Katalysatoraktivität kann diese durch Regenerierung mit geeigneten Mittel (Oxidationsmittel wie Sauerstoff, unter Beimischung eines geeigneten Verdünnungsmittel wie Stickstoff oder HF) vollständig wiederhergestellt werden. Diese Regenerierung kann mehrfach durchgeführt werden, ohne daß eine erkennbare Schädigung des Katalysators auftritt.

Das erfindungsgemäße Verfahren kann beispielsweise so durchgeführt werden, daß die Ausgangsstoffe Perchlorethylen und Fluorwasserstoff kontinuierlich in einen Verdampfer aus Edelstahl oder Nickel gefördert werden. Die Verdampfertemperatur muß ausreichen, um das gesamte Reaktionsgemisch zu verdampfen, ist aber ansonsten für den Ablauf der Reaktion nicht kritisch. Die gasförmigen Edukte gelangen über eine beheizte Strecke in einen Gasmischer und dann in den Reaktor. Der Gasmischer kann sich auch im unteren Teil des Reaktors, der die Schüttung des Katalysators enthält, befinden.

Das erfindungsgemäße Verfahren kann in einem einzelnen Reaktor oder auch in mehreren hintereinandergeschalteten Reaktoren (Kaskade) durchgeführt werden. Die Abtrennung des bei der Reaktion gebildeten Chlorwasserstoffs kann bei Verwendung einer Kaskade nach Durchlauf des letzten Reaktors erfolgen (einstufige Verfahrensweise) oder man trennt einen Teil des Chlorwasserstoffs bereits nach einem oder mehreren der dem letzten vorhergehenden Reaktoren ab und den Rest nach dem letzten Reaktor (mehrstufige Verfahrensweise).

Der Reaktor bzw. die Reaktoren sind aus einem beständigen Material wie Edelstahl, Nickel oder Hastelloy gefertigt. Die Reaktorform kann aus verschiedenen technischen Ausführungen wie Rohrreaktor, Ringspaltreaktor oder Schachtreaktor ausgewählt werden.

Durch Beheizung von außen wird in der Reaktionszone eine Temperatur von 150 bis 450°C, bevorzugt von 180 bis 370°C, insbesondere 200 bis 350°C gehalten. Bei Einsatz einer Kaskade ist es sinnvoll, im ersten und im letzten Reaktor eine niedrigere Temperatur aus den angegebenen Bereichen zu wählen als in dem oder den zwischen ihnen liegenden Reaktoren.
Um einen möglichst vollständigen Umsatz des Perchlorethylens zu erreichen, wird der Fluorwasserstoff bevorzugt im Überschuß eingesetzt. Das Molverhältnis HF:Perchlorethylen ist vorzugsweise 3:1 bis 10:1, insbesondere 4:1 bis 8:1.

Die erfindungsgemäße Gasphasenreaktion ist unter erhöhtem Druck besonders effektiv, da der hohe Umsatz dann über einen längeren Zeitraum erhalten bleibt. Der Druck wird während der Reaktion über ein Regelventil auf 1 bis 26 bar, bevorzugt 2 bis 17 bar, besonders bevorzugt 4 bis 10 bar eingestellt.
Bei kontinuierlichem Betrieb umfaßt die Aufarbeitung des Reaktiongemisches vorteilhafterweise die Abtrennung des während des Reaktordurchgangs gebildeten Chlorwasserstoffs, des Zielprodukts R 125 und der hochfluorierten Nebenprodukte sowie die Rückspeisung des Restgases, das mit frischem Fluorwasserstoff und Perchlorethylen angereichert wird, in den Reaktionsteil. Die Edukte werden in technischer Reinheit verwendet. Insbesondere wird zweckmäßigerweise die Restwassermenge minimiert. Die Ausgangsverbindungen sind nach bekannten Verfahren in einfacher Weise herstellbar.
Das erfindungsgemäße Verfahren wird durch die nachfolgenden Beispiele näher erläutert. Die angegebenen Prozente sind der GC-Analyse sind Flächenprozente.

### Beispiele

Cr(NO₃)₃•9H₂O wurde in Wasser gelöst. Diese Lösung wurde zu einer Mischung von Magnesiumoxid und Graphit gegeben und die dabei entstehende pastöse Masse innig verknetet. Anschließend wurde das pastöse Produkt granuliert und 20 Stunden bei 100°C getrocknet. Die bei den verschiedenen Katalysatorchargen eingesetzten Substanzmengen sind der Tabelle 1 zu entnehmen.
Die Aktivierung der getrockneten Katalysatoren A bis E erfolgte vor Durchführung des jeweiligen Tests in dem im Vergleichsbeispiel beschriebenen Reaktorrohr durch Behandlung mit einem HF/Stickstoff-Gemisch bei Temperaturen zwischen 200 und 500°C. Diese Behandlung dauerte 6 bis 15 h.

**Tabelle 1**

| Katalysator | A | B | C | D | E |
|---|---|---|---|---|---|
| Gehalt an Cr (Gew.-%), ausgedrückt als Cr₂O₃ | 4,3 | 5,1 | 16,0 | 23,3 | 26,3 |
| Gehalt an Mg (Gew.-%), ausgedrückt als MgO | 82,4 | 67,8 | 60,0 | 53,7 | 51,6 |
| Gehalt an Graphit (Gew.-%) | 13,2 | 27,1 | 24,0 | 23,0 | 22,1 |

| Einsatzmengen [g]: | | | | | |
|---|---|---|---|---|---|
| Cr(NO₃)₃•9H₂O | 310 | 400 | 2800 | 800 | 940 |
| MgO | 1120 | 1000 | 2000 | 350 | 350 |
| Graphit | 180 | 400 | 800 | 150 | 150 |
| Wasser | 450 | 600 | 1200 | 1200 | 1500 |

### Vergleichsbeispiel

Perchlorethylen und Fluorwasserstoff wurden im angegebenen Molverhältnis (Tabelle 2) vermischt, über beheizte Leitungen einem Verdampfer zugeführt und im gasförmigen Zustand über die Schüttung des Katalysators A in einem Rohrreaktor aus Nickel geleitet (⌀ = 42 mm). Die eingefüllte Menge an nichtaktiviertem Katalysator betrug 0,5 l Schüttvolumen vor der Aktivierung. Der Reaktor wurde elektrisch beheizt. Die angegebene Reaktionstemperatur (Tabelle 2) ist die gemittelte Temperatur im Inneren der Katalysatorschüttung. Die den Reaktor verlassenden gasförmigen Produkte wurden durch eine Wasserwäsche geleitet. Einer Trocknung mit CaCl₂ folgte die Probenahme aus dem Produktgastrom. Die Proben wurden gaschromatographisch analysiert. Die ermittelte Zusammensetzung des Produktgasstroms ist in Tabelle 2 angegeben.

### Beispiele 1 bis 5

Die Katalysatoren B, C, D, E wurden genauso wie Katalysator A unter den in Tabelle 2 angegebene Bedingungen getestet. Die Resultate sind in derselben Tabelle angegeben. Katalysator C wurde einmal bei 320°C (Beispiel 2) und einmal bei 295°C (Beispiel 5) getestet.

| Beispiel Nr. | | Vergleichsbeispiel | 1 | 2 | 3 | 4 | 5 |
|---|---|---|---|---|---|---|---|
| Katalysator | | A | B | C | D | E | C |
| Gehalt an Cr, ausgedrückt als Cr₂O₃ [Gew.-%] | | 4,3 | 5,1 | 16,0 | 23,3 | 26,3 | 16,0 |
| Molverhältnis HF/Perchlorethylen | | 5,1/1 | 5,0/1 | 5,2/1 | 5,4/1 | 5,3/1 | 5,1/1 |
| Druck [bar abs.] | | 1 | 1 | 1 | 1 | 1 | 1 |
| Verweilzeit [s] | | 10 | 11 | 10 | 9 | 10 | 10 |
| Reaktionstemperatur [°C] | | 320 | 320 | 320 | 320 | 320 | 295 |
| Umsatz des Perchlorethylens [%] | | 38,7 | 75,9 | 93,0 | 77,6 | 60,1 | 89,0 |
| Wertstoffselektivität [%] | | 97,4 | 97,5 | 97,5 | 97,2 | 95,0 | 97,9 |
| erhaltene Produkte [%] | R 125 | 8,2 | 20,4 | 34,4 | 27,0 | 20,1 | 23,4 |
| | R 124s | 16,4 | 27,7 | 18,4 | 20,1 | 17,0 | 25,4 |
| | R 123s | 12,5 | 25,0 | 36,2 | 27,9 | 19,6 | 35,3 |
| | R 122s | 0,6 | 0,9 | 1,7 | 0,4 | 0,4 | 3,0 |
| | R 121s | Spuren | Spuren | Spuren | Spuren | Spuren | Spuren |
| | R 1111 | 400 ppm | 200 ppm | 200 ppm | 300 ppm | 300 ppm | 150 ppm |
| | R 1112a | 700 ppm | 60 ppm | 100 ppm | 180 ppm | 180 ppm | 80 ppm |
| | Σ_{R 130er} | 0,4 | 0,9 | 1,0 | 1,1 | 1,4 | 0,6 |
| | Σ_{R 110er} | 0,4 | 0,9 | 1,0 | 1,0 | 1,4 | 0,6 |
| s = Summe für alle vorkommenden Konstitutionsisomeren gleicher Summenformel (Beispiel: R 123s = R 123, R 123a und R 123b) Σ_{R 110er} = Summe für alle Verbindungen, denen eine Zahlenkombination zwischen 110 und 120 zugeordnet ist (R 111, R 112, R 113, R 114 und ihre Konstitutionsisomeren, sowie R 115 und R 116). Diese Regel ist sinngemäß für die R 130er-Reihe anzuwenden Wertstoffselektivität = Selektivität zu R 125 + R 124s + R 123s + R 122s + R 121s | | | | | | | |

## Patentansprüche

1. Verfahren zur Herstellung von Pentafluorethan (R 125) durch Reaktion von Perchlorethylen mit Fluorwasserstoff in der Gasphase an einem chromhaltigen Katalysator, dadurch gekennzeichnet, daß ein Chrom und Magnesium enthaltender Katalysator verwendet wird, der dadurch erhältlich ist, daß man ein wasserlösliches Chrom(III)-Salz mit Magnesiumhydroxid oder Magnesiumoxid sowie optional Graphit in Gegenwart von Wasser umsetzt, die Reaktionsmischung in einen Teig überführt, dann den Teig trocknet und bei Temperaturen von 20 bis 500°C mit Fluorwasserstoff behandelt, wobei man solche Mengen an Chrom(III)- und Magnesiumsalzen sowie optional Graphit verwendet, daß der getrocknete Teig vor der Behandlung mit Fluorwasserstoff 4,5 bis 26 Gew.-% Cr, ausgedrückt als Cr₂O₃, und mindestens 25 Gew.-% Mg, ausgedrückt als MgO, sowie optional Graphit enthält.

2. Verfahren nach Anspruch 1, dadurch gekennzeichnet, daß man solche Mengen an Chrom(III)- und Magnesiumsalzen sowie optional Graphit verwendet, daß der getrocknete Teig vor der Behandlung mit Fluorwasserstoff 5,5 bis 23 Gew.-% Cr, ausgedrückt als Cr₂O₃, und mindestens 25 Gew.-% Mg, ausgedrückt als MgO, sowie optional Graphit enthält.

3. Verfahren nach Anspruch 1 oder 2, dadurch gekennzeichnet, daß der getrocknete Teig vor der Behandlung mit Fluorwasserstoff 5 bis 40 Gew.-% Graphit enthält.

4. Verfahren nach einem der Ansprüche 1 bis 3, dadurch gekennzeichnet, daß man die Reaktion von Perchlorethylen mit Fluorwasserstoff bei einer Temperatur von 150 bis 450°C durchführt.

5. Verfahren nach einem der Ansprüche 1 bis 4, dadurch gekennzeichnet, daß man die Reaktion von Perchlorethylen mit Fluorwasserstoff bei einem Druck von 2 bis 17 bar durchführt.

6. Verfahren nach einem der Ansprüche 1 bis 4, dadurch gekennzeichnet, daß man die Reaktion von Perchlorethylen mit Fluorwasserstoff bei einem Druck von 4 bis 10 bar durchführt.

7. Verfahren nach einem der Ansprüche 1 bis 6, dadurch gekennzeichnet, daß man die Reaktion von Perchlorethylen mit Fluorwasserstoff bei einem Molverhältnis HF:Perchlorethylen von 3:1 bis 10:1 durchführt.

8. Verfahren zur Herstellung von Pentafluorethan (R 125) durch Reaktion von Perchlorethylen mit Fluorwasserstoff in der Gasphase an einem chromhaltigen Katalysator, dadurch gekennzeichnet, daß ein Chrom und Magnesium sowie optional Graphit enthaltender Katalysator verwendet wird, und daß man solche Mengen an Chrom(III)- und Magnesiumsalzen sowie optional Graphit verwendet, daß der Katalysator 4,5 bis 26 Gew.-% Cr, ausgedrückt als Cr₂O₃, und mindestens 25 Gew.-% Mg, ausgedrückt als MgO, sowie optional Graphit enthält.

9. Verfahren nach Anspruch 8, dadurch gekennzeichnet, daß man solche Mengen an Chrom(III)- und Magnesiumsalzen sowie optional Graphit verwendet, daß der Katalysator 5,5 bis 23 Gew.-% Cr, ausgedrückt als Cr₂O₃, und mindestens 25 Gew.-% Mg, ausgedrückt als MgO, sowie optional Graphit enthält.

10. Verfahren nach Anspruch 8 oder 9, dadurch gekennzeichnet, daß der Katalysator 5 bis 40 Gew.-% Graphit enthält.

11. Verfahren nach einem der Ansprüche 8 bis 10, dadurch gekennzeichnet, daß man die Reaktion von Perchlorethylen mit Fluorwasserstoff bei einer Temperatur von 150 bis 450°C durchführt.

12. Verfahren nach einem der Ansprüche 8 bis 11, dadurch gekennzeichnet, daß man die Reaktion von Perchlorethylen mit Fluorwasserstoff bei einem Druck von 2 bis 17 bar durchführt.

13. Verfahren nach einem der Ansprüche 8 bis 11, dadurch gekennzeichnet, daß man die Reaktion von Perchlorethylen mit Fluorwasserstoff bei einem Druck von 4 bis 10 bar durchführt.

14. Verfahren nach einem der Ansprüche 8 bis 13, dadurch gekennzeichnet, daß man die Reaktion von Perchlorethylen mit Fluorwasserstoff bei einem Molverhältnis HF:Perchlorethylen von 3:1 bis 10:1 durchführt.

## Claims

1. A process for preparing pentafluoroethane (R 125) by reacting perchloroethylene with hydrogen fluoride in the gas phase over a chromium-containing catalyst, wherein the catalyst used comprises chromium and magnesium and is obtainable by reacting a watersoluble chromium(III) salt with magnesium hydroxide or magnesium oxide and, if desired, graphite in the presence of water, converting the reaction mixture into a paste, then drying the paste and treating it with hydrogen fluoride at temperatures of from 20 to 500°C, using such amounts of chromium and magnesium salts and, if desired, graphite that the dried paste before treatment with hydrogen fluoride contains from 4.5 to 26% by weight of Cr, expressed as Cr₂O₃, and at least 25% by weight of Mg, expressed as MgO, and, if desired, graphite.

2. The process as claimed in claim 1, wherein such amounts of chromium(III) and magnesium salts and, if desired, graphite are used that the dried paste before treatment with hydrogen fluoride contains from 5.5 to 23% by weight of Cr, expressed as Cr₂O₃, and at least 25% by weight of Mg, expressed as MgO, and, if desired, graphite.

3. The process as claimed in claim 1 or 2, wherein the dried paste before treatment with hydrogen fluoride contains from 5 to 40% by weight of graphite.

4. The process as claimed in any of claims 1 to 3, wherein the reaction of perchloroethylene with hydrogen fluoride is carried out at a temperature of from 150 to 450°C.

5. The process as claimed in any of claims 1 to 4, wherein the reaction of perchloroethylene with hydrogen fluoride is carried out at a pressure of from 2 to 17 bar.

6. The process as claimed in any of claims 1 to 4, wherein the reaction of perchloroethylene with hydrogen fluoride is carried out at a pressure of from 4 to 10 bar.

7. The process as claimed in any of claims 1 to 6, wherein the reaction of perchloroethylene with hydrogen fluoride is carried out at a molar ratio HF:perchloroethylene of from 3:1 to 10:1.

8. A process for preparing pentafluoroethane (R 125) by reacting perchloroethylene with hydrogen fluoride in the gas phase over a chromium-containing catalyst, wherein the catalyst used comprises chromium and magnesium and, if desired, graphite and the amounts of chromium(III) and magnesium salts and, if desired, graphite used are such that the catalyst contains from 4.5 to 26% by weight of Cr, expressed as Cr₂O₃, and at least 25% by weight of Mg, expressed as MgO, and, if desired, graphite.

9. The process as claimed in claim 8, wherein the amounts of chromium(III) and magnesium salts and, if desired, graphite used are such that the catalyst contains from 5.5 to 23% by weight of Cr, expressed as Cr₂O₃, and at least 25% by weight of Mg, expressed as MgO, and, if desired, graphite.

10. The process as claimed in claim 8 or 9, wherein the catalyst contains from 5 to 40% by weight of graphite.

11. The process as claimed in any of claims 8 to 10, wherein the reaction of perchloroethylene with hydrogen fluoride is carried out at a temperature of from 150 to 450°C.

12. The process as claimed in any of claims 8 to 11, wherein the reaction of perchloroethylene with hydrogen fluoride is carried out at a pressure of from 2 to 17 bar.

13. The process as claimed in any of claims 8 to 11, wherein the reaction of perchloroethylene with hydrogen fluoride is carried out at a pressure of from 4 to 10 bar.

14. The process as claimed in any of claims 8 to 13, wherein the reaction of perchloroethylene with hydrogen fluoride is carried out at a molar ratio HF: perchloroethylene of from 3:1 to 10:1.

## Revendications

1. Procédé pour la préparation de pentafluoroéthane (R 125) par réaction de perchloroéthylène avec du fluorure d'hydrogène en phase gazeuse sur un catalyseur contenant du chrome, caractérisé en ce qu'on utilise un catalyseur contenant du chrome et du magnésium qui peut être obtenu par réaction d'un sel de chrome (III) hydrosoluble avec de l'hydroxyde de magnésium ou de l'oxyde de magnésium, ainsi que facultativement du graphite en présence d'eau, par transformation du mélange réactionnel en pâte, par séchage subséquent de la pâte et par traitement à des températures de 20 à 500 °C avec du fluorure d'hydrogène, en utilisant des quantités de sels de chrome (III) et de magnésium, ainsi que facultativement de graphite telles, que la pâte séchée contient, avant le traitement au fluorure d'hydrogène, 4,5 à 26 % en poids de Cr, exprimé sous forme de Cr₂O₃, et au moins 25 % en poids de Mg, exprimé sous forme de MgO, ainsi que facultativement du graphite.

2. Procédé selon la revendication 1, caractérisé en ce qu'on utilise des quantités de sels de chrome (III) et de magnésium, ainsi que facultativement de graphite telles, que la pâte séchée contient, avant le traitement au fluorure d'hydrogène, 5,5 à 23 % en poids de Cr, exprimé sous forme de Cr₂O₃, et au moins 25 % en poids de Mg, exprimé sous forme de MgO, ainsi que facultativement du graphite.

3. Procédé selon la revendication 1 ou 2, caractérisé en ce que la pâte séchée contient, avant le traitement au fluorure d'hydrogène, 5 à 40 % en poids de graphite.

4. Procédé selon l'une des revendications 1 à 3, caractérisé en ce qu'on effectue la réaction de perchloroéthylène avec le fluorure d'hydrogène à une température de 150 à 450 °C.

5. Procédé selon l'une des revendications 1 à 4, caractérisé en ce qu'on effectue la réaction de perchloroéthylène avec le fluorure d'hydrogène à une pression de 2 à 17 bar.

6. Procédé selon l'une des revendications 1 à 4, caractérisé en ce qu'on effectue la réaction de perchloroéthylène avec le fluorure d'hydrogène à une pression de 4 à 10 bar.

7. Procédé selon l'une des revendications 1 à 6, caractérisé en ce qu'on effectue la réaction de perchloroéthylène avec le fluorure d'hydrogène à un rapport molaire de HF : perchloroéthylène de 3 : 1 à 10 : 1.

8. Procédé pour la préparation de pentafluoroéthane (R 125) par réaction de perchloroéthylène avec du fluorure d'hydrogène en phase gazeuse sur un catalyseur contenant du chrome, caractérisé en ce qu'on utilise un catalyseur contenant du chrome et du magnésium ainsi que facultativement du graphite et en ce qu'on utilise des quantités de sels de chrome (III) et de magnésium, ainsi que facultativement de graphite telles, que le catalyseur contient 4,5 à 26 % en poids de Cr, exprimé sous forme de Cr₂O₃, et au moins 25 % en poids de Mg, exprimé sous forme de MgO, ainsi que facultativement du graphite.

9. Procédé selon la revendication 8, caractérisé en ce qu'on utilise des quantités de sels de chrome (III) et de magnésium, ainsi que facultativement de graphite telles, que le catalyseur contient 5,5 à 23 % en poids de Cr, exprimé sous forme de Cr₂O₃, et au moins 25 % en poids de Mg, exprimé sous forme de MgO, ainsi que facultativement du graphite.

10. Procédé selon la revendication 8 ou 9, caractérisé en ce que le catalyseur contient 5 à 40 % en poids de graphite.

11. Procédé selon l'une des revendications 8 à 10, caractérisé en ce qu'on effectue la réaction de perchloroéthylène avec le fluorure d'hydrogène à une température de 150 à 450 °C.

12. Procédé selon l'une des revendications 8 à 11, caractérisé en ce qu'on effectue la réaction de perchloroéthylène avec le fluorure d'hydrogène à une pression de 2 à 17 bar.

13. Procédé selon l'une des revendications 8 à 11, caractérisé en ce qu'on effectue la réaction de perchloroéthylène avec le fluorure d'hydrogène à une pression de 4 à 10 bar.

14. Procédé selon l'une des revendications 8 à 13, caractérisé en ce qu'on effectue la réaction de perchloroéthylène avec le fluorure d'hydrogène à un rapport molaire de HF : perchloroéthylène de 3 : 1 à 10 : 1.
